# EUROPÄISCHE PATENTSCHRIFT

(11) **EP 1 062 906 B1**
(45) Veröffentlichungstag und Bekanntmachung des Hinweises auf die Patenterteilung: **11.04.2007**
(21) Anmeldenummer: 00112337.1
(22) Anmeldetag: 09.06.2000
(51) Int. Cl.: A61B 5/00, G06F 19/00

(54) **Vorrichtung zur medizinischen Langzeitüberwachung von Personen**
Device for medical long term supervision of persons
Dispositif de surveillance médicale à long terme appliqué à des personnes

(30) Priorität: 26.06.1999 DE 19929328
(43) Veröffentlichungstag der Anmeldung: 27.12.2000
(73) Patentinhaber: Astrium GmbH, 85521 Ottobrunn (DE)
(72) Erfinder: Rode, Wilfried, 27367 Sottrum (DE); Klintworth, Rolf, Dr., 27751 Delmenhorst (DE); Ludwig, Klaus-Peter, 88662 Überlingen (DE); Oberle, Michael, 8051 Zürich (CH)
(74) Vertreter: Hansmann, Dierk

(56) Entgegenhaltungen:
- EP-A- 0 540 154
- WO-A-91/00054
- WO-A-98/43537
- PATENT ABSTRACTS OF JAPAN vol. 018, no. 131 (C-1175), 3. März 1994 (1994-03-03) & JP 05 317278 A (SUZUKEN:KK), 3. Dezember 1993 (1993-12-03)
- PATENT ABSTRACTS OF JAPAN vol. 1997, no. 08, 29. August 1997 (1997-08-29) & JP 09 089676 A (CASIO COMPUT CO LTD), 4. April 1997 (1997-04-04)

## Beschreibung

Die Erfindung betrifft eine Vorrichtung zur medizinischen Langzeitüberwachung von Personen zur medizinischen Betreuung mit auf der Körperoberfläche angeordneten Sensoren sowie einer diesen zugeordneten Datenaufzeichnungseinheit für einen Datenaustausch.

Die an Bord von Weltraumstationen, insbesondere auch der Internationalen Raumstation ISS eingesetzten Astronauten müssen über einen längeren Zeitraum unter extremsten äußeren Bedingungen leben und arbeiten. Zu ihrem persönlichen Schutz sowie im Hinblick auf den Erfolg der gesamten Mission ist daher eine kontinuierliche medizinische Betreuung erforderlich.

Das hierbei eingesetzte Monitorsystem muß klein, portabel und von einem Astronauten allein zu bedienen sein. Die Überwachung muß an jedem Ort innerhalb der Raumstation sowie, bei sogenannten Extravehicular Activities (EVAs), auch außerhalb dieser gewährleistet sein. Zudem muß ein derartiges System möglichst einfach in die bereits bestehende Telekommunikations-Infrastruktur einer Raumstation zu integrieren sein.

Auch außerhalb der Raumfahrtmedizin wird eine zunehmende Miniaturisierung von medizinischen Geräten, wie Elektrokardiographen (EKG) oder Pulsoximetern, angestrebt. Dies dient nicht nur dem Ziel größtmöglicher Portabilität, wie sie beispielsweise in der Notfallmedizin erforderlich ist, sondern, beispielsweise im Sport- und Freizeitbereich, der Überwachung der allgemeinen Fitneß oder der aktuellen körperlichen Belastung.

Zwar existiert zu diesem Zweck bereits eine Reihe von Geräten, insbesondere in Form portabler Transientenrekorder, doch beschränken sich diese Systeme im wesentlichen auf die Erfassung weniger biomedizinischer Signale. Die Anzahl der eingesetzten Sensoren ist vergleichsweise begrenzt, und die Signalübertragung vom Sensor zu einem angeschlossenen Datenlogger sowie zu möglicherweise vorgesehenen nachgeordneten Prozeßrechnern erfolgt in der Regel drahtgebunden. Sollen weitere Signale gemessen werden, so wächst die Gerätegröße entsprechend an oder es muß ein zusätzlicher Rekorder eingesetzt werden. Daher sind diese bekannten Geräte für ein umfassendes, ortsunabhängiges und benutzerfreundliches Telemonitoring, das zudem flexibel auf veränderte Ansprüche reagieren soll, nur bedingt geeignet.

Ferner ist gemäß DE 196 07 222 A1 bekannt, einen implantierbaren miniturisierten Sensor zur Speicherung von Patientendaten und der Aufnahme von Biopotentialen vorzusehen.

Nach der Veröffentlichung Patent Abstracts of Japan vol. 018, no. 131 (C-1175), 3. März 1994 (1994-03-03) & JP 05 317278 A (Suzuken:KK), 3. Dezember 1993 (1993-12-03) ist eine Vorrichtung bekannt geworden, bei der körperliche Meßdaten über eine Funkelemetrie vom Meßort übertragen werden. Hierbei dient der menschliche Körper als Antenne, wobei die Antennen von Sende- und

Empfangseinheit so abgestimmt sind, daß sie nur mit einem Körper in unmittelbarer Nähe funktionieren.

Aufgabe der Erfindung ist es deshalb, eine Vorrichtung medizinischen Langzeitüberwachung der eingangs genannten Art bereitzustellen, die den flexiblen Einsatz unterschiedlicher, autonomer Sensoren an beliebigen Stellen des Körpers zur gleichen Zeit ermöglicht, die als umfassendes Monitoring-System in bestehende Infrastrukturen integriert werden kann und die für einen Einsatz in der Fernüberwachung mittels Satellitenkommunikation geeignet ist.

Die Erfindung löst diese Aufgabe durch die Merkmale des Anspruches 1.

Die Sensordaten werden dabei ohne zusätzliche Kabelverbindung direkt über die Haut ausgetauscht. Dies hat den Vorteil, daß die Sensoren an jeder Stelle des Körpers automatisch an den dazugehörigen elektrischen Leiter zur Signalübertragung angeschlossen sind. Die köpergebundene Datenübertragung hat zudem den Vorteil, daß die hierzu notwendige Sendeleistung äußerst gering ist. Dies vermeidet die Störung anderer Geräte und behindert das unerlaubte Abhören sensitiver medizinischer Daten. Der daraus weiterhin resultierende geringere Leistungsverbrauch unterstützt zusätzlich das Ziel einer Langzeitüberwachung.

Dadurch, daß in weiterer Ausgestaltung der Erfindung bei der erfindungsgemäßen Vorrichtung hochminiaturisierte Komponenten verwendet werden, werden zugleich der Tragekomfort und die Benutzerfreundlichkeit wesentlich verbessert. Ferner können mehrere Sensoren gleichzeitig eingesetzt werden, ohne daß sich der Tragekomfort verringert. Das System erlaubt ein einfaches und schnelles Entfernen oder Hinzufügen von Sensoren. Eine weitere zentrale Sende-und Empfangseinheit, ein sogenannter Body-Transceiver, oder ein veränderter Systemaufbau sind hierzu nicht erforderlich. Durch eine gezielte Signalvorverarbeitung innerhalb der Sensoren und die Verwendung eines einheitlichen Interfaces zum anschließenden Datenaustausch über die Haut ist es zudem möglich, völlig unterschiedliche Sensoren nebeneinander einzusetzen.

Bei einer Verwendung der erfindungsgemäßen Grundkonzeption aus Sensoren, Body-Transceiver und Datenlogger können die Daten entweder auf Speicherkarten geschrieben oder aber über eine Schnittstelle in einen PC geladen werden. Die Datenübertragung an den Datenlogger geschieht per Telemetrie; die Reichweite ist auf wenige Meter begrenzt. Das Gerät selbst kann am Gürtel oder in der Tasche getragen werden.

Die erfindungsgemäße Vorrichtung kann zusätzlich in lokale Monitoringsysteme von Spitälern oder Raumstationen integriert werden. Im Falle von Raumstationen ermöglicht sie eine globale Überwachung unter Ausnutzung bestehender Satellitenkommunikationssysteme, wobei entsprechende Hochfrequenz- oder Infrarot- Sende- und Empfangseinheiten in den Datenlogger oder in den Body-Transceiver integriert sein können.

In erster Linie können Sensoren zur Messung von Parametern wie beispielsweise der Temperatur, des EKG, des Pulses, der Sauerstoffsättigung oder der Hautleitfähigkeit Verwendung finden. Darüber hinaus können diese aber auch mit sogenannten Umweltsensoren kombiniert werden, die beispielsweise die vorhandene Sauerstoffqualität, die Umgebungstemperatur oder auch den Aufenthaltsort mittels eines Global-Positioning-Systems (GPS) erfassen.

Mithilfe der erfindungsgemäßen Vorrichtung kann der Benutzer schnell und flexibel auf veränderte Beobachtungsaufgaben reagieren. Wie ein herkömmliches Pflaster können die Sensoren ohne zusätzliche Hilfe an jeder Stelle des Körpers hinzugefügt oder entfernt werden. Um diese Mobilität und Miniaturisierung zu erreichen, werden Mikrochips mit sensorspezifischer Signalverarbeitung und einheitlicher Datenübertragung direkt am jeweiligen Sensor angebracht. Die Vorrichtung nach der Erfindung ist somit in hohem Masse flexibel und bedienungsfreundlich und erlaubt zudem den Einsatz neuartiger Sensoren, ohne gleichzeitig Modifikationen der anderen Module durchführen zu müssen.

Die erfindungsgemäße Vorrichtung eignet sich außer für einen Einsatz in der Raumfahrttechnik in besonderem Maße für Patienten, die sich in ambulanter Behandlung befinden und eine weitere Überwachung benötigen und die auf diese Weise unabhängig von der vorhandenen Telekommunikations-Infrastruktur werden. Sie ist weiterhin für alleinstehende, chronisch kranke oder behinderte Menschen sowie für die Sport- und Arbeitsmedizin geeignet. In Krankenhäusern ermöglicht die Vorrichtung nach der Erfindung ein kabelloses Patienten-Monitoring in Krankenzimmern, Fluren und im Operationssaal sowie eine Integration in eine elektronische Patientendatenerfassung. Bei Rettungsdiensten und Katastropheneinsätzen schließlich eignet sich die erfindungsgemäße Vorrichtung zur kontinuierlichen Überwachung von Verletzten nach der Erstbehandlung durch den Notarzt sowie für mobile Krankenhäuser, die mit einem Minimum an Geräten ausgestattet und auf einen Datenaustausch mit medizinischen Experten via Satellit angewiesen sind.

Nachfolgend soll die Erfindung anhand von in der Zeichnung dargestellten Ausführungsbeispielen näher erläutert werden.

Es zeigen:
- Fig. 1: einen Mikrosensor in perspektivischer Darstellung,
- Fig. 2: eine vergrößerte Darstellung der Anordnung gemäß Fig. 1,
- Fig. 3: den Einsatz einer Vorrichtung zur medizinischen Langzeitüberwachung gemäß den Figuren 1 und 2 bei einem Astronauten und
- Fig. 4: eine Übersicht über ein Gesamtsystem zur medizinischen Fernüberwachung in schematischer Darstellung.

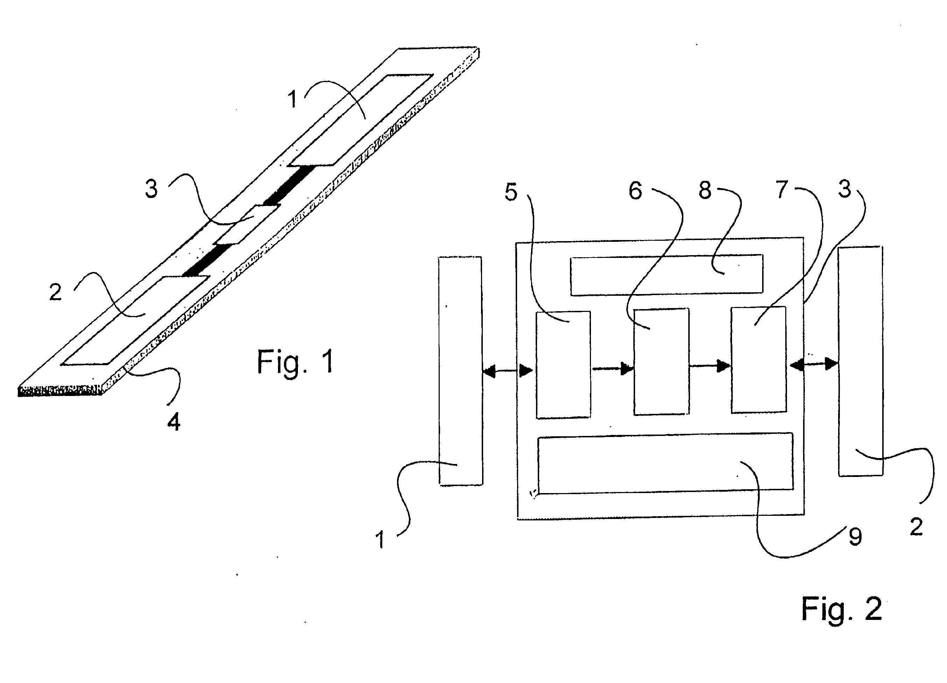

Miniaturisierte Sensoren bzw. Elektroden 1 sind zusammen den Sende-/Empfangselektroden 2 sowie einem Mikrochip 3 auf einem Sensorträger 4 angeordnet, der wie ein Pflaster auf die Haut geklebt werden kann. Der Mikrochip 3 stellt ein hochgradig integriertes Bauteil dar und umfaßt, wie aus der Detaildarstellung in Fig. 2 ersichtlich ist eine dem Sensor 1 nachgeschaltete Meßdatenerfassungseinheit 5, eine Signalvorverarbeitungseinheit 6, sowie einen Transceiver 7, der mit den Sende-/Empfangselektroden 2 verbunden ist. Zusätzlich umfaßt der Mikrochip 3 eine Batterie 8 sowie eine Kontroll- und Überwachungseinheit 9 für die Spannungsversorgung.

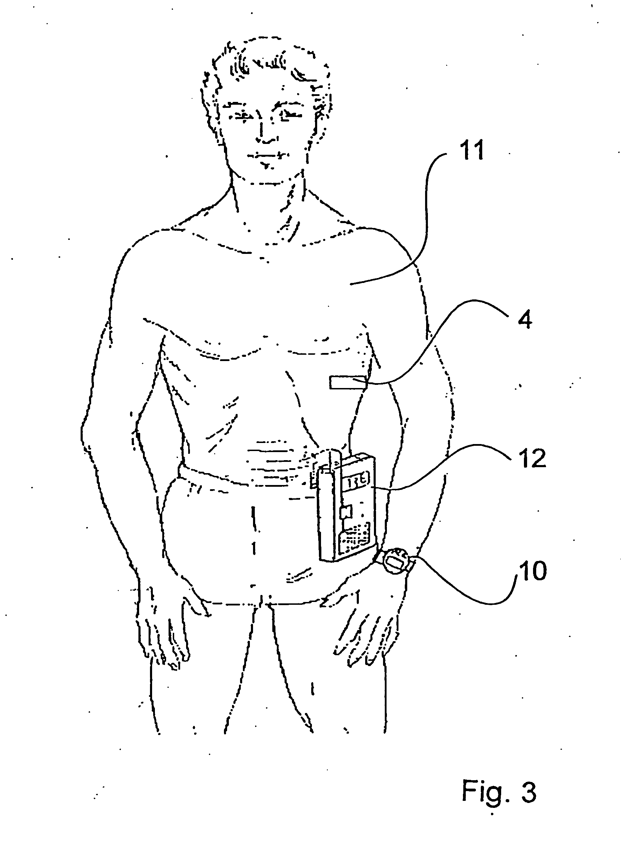

Wie in Fig. 3 gezeigt, können eine oder mehrere derartige Sensorträger 4 am Körper eines Astronauten angebracht sein, indem sie wie ein Pflaster auf dessen Haut geklebt werden. Die von den Sensoren 1 aufgenommenen Signale werden direkt im nachgeschalteten Mikrochip 3 verarbeitet, und das Ergebnis wird anschließend drahtlos an einen sogenannten Body-Transceiver 10 weitergeleitet. Dieser wird wie eine Armbanduhr vom Astronauten 11 getragen. Der Datenaustausch erfolgt dabei unmittelbar über die Haut/Gewebe und ist somit an den Körper gebunden.

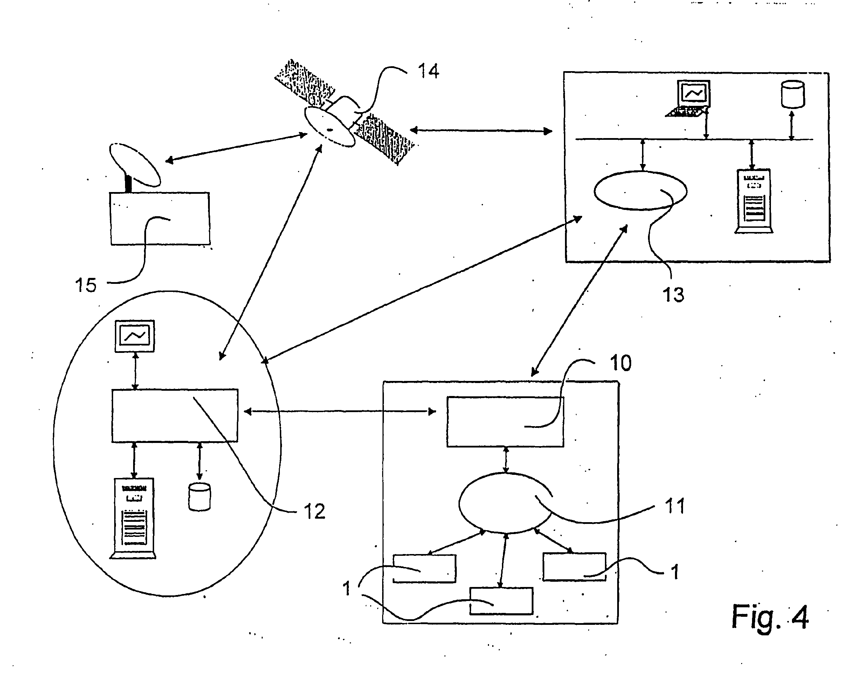

Zusätzlich zum Body-Transceiver 10 trägt der Astronaut 11 in einer Gürteltasche einen Datenlogger 12, in dem die vom Body-Transceiver 10 übermittelten Daten gespeichert werden.
Hier können sie entweder zu einem späteren Zeitpunkt entnommen und zur nachträglichen Auswertung ausgelesen bzw. graphisch dargestellt werden, oder aber die Daten werden unmittelbar, wie dies abschießend in Fig. 4 dargestellt ist, auf drahtlosem Wege mittels Hochfrequenz- oder Infrarotübertragung weitergeleitet. Die Übertragung kann dabei unmittelbar vom Body-Transceiver 10 selbst oder vom Datenlogger zu einer Basisstation 13, beispielsweise der Raumstation, erfolgen. Sie kann aber auch unter Zwischenschaltung eines Satelliten 14 entweder zur Basisstation 13 oder zu einem Hospital 15 geschehen. Letzteres gilt insbesondere für rein terrestrische Anwendungen der Vorrichtung nach der Erfindung.

## Patentansprüche

1. Vorrichtung zur medizinischen Langzeitüberwachung von Personen zur medizinischen Betreuung mit auf der Körperoberfläche angeordneten Sensoren (1) sowie einer diesen zugeordneten Datenaufzeichnungseinheit (12) für einen Datenaustausch, wobei die Vorrichtung aus wenigstens einer autonomen Sensoreinheit (1 - 3), die Sende-und Empfangselektroden (2) und einen der Sensoren (1) aufweist, einer zentralen Sende- und Empfangseinheit (10) sowie einer portablen Datenaufzeichnungseinheit (12) besteht, wobei die zentrale Sende- und Empfangseinheit (10) am Körper zugeordnet ist, die Sensoreinheit (1 - 3) einen Mikrochip (3) enthält, der die vom Sensor (1) aufgenommenen Signale in Sensordaten verarbeitet, die Sensordaten zwischen der Sensoreinheit (1 - 3) und der zentralen Sende- und Empfangseinheit (10) unmittelbar über die Haut oder das Gewebe und somit körpergebunden austauschbar sind, die Sensoreinheit (1 - 3) auf einem Sensorträger (4) angeordnet ist, der als Pflaster ausgebildet ist, und der Mikrochip (3) eine dem Sensor (1) nachgeschaltete Meßdatenerfassungseinheit (6) sowie einen Transceiver (7) umfaßt, der mit den Sende-und Empfangselektroden (2) verbunden ist.

2. Vorrichtung nach Anspruch 1, **dadurch gekennzeichnet, daß** der Mikrochip (3) eine Batterie (8) sowie eine Kontroll- und Überwachungseinheit (9) für die Spannungsversorgung umfaßt.

3. Vorrichtung nach Anspruch 1 oder 2, **dadurch gekennzeichnet, daß** die portable Datenaufzeichnungseinheit aus einem Datenlogger (12) besteht.

4. Vorrichtung nach Anspruch 3, **dadurch gekennzeichnet, daß** die Datenübertragung zwischen der zentralen Sende-und Empfangseinheit (10) und dem Datenlogger (12) mittels Telemetrie erfolgt.

## Claims

1. Device for medically monitoring persons over a long period to provide medical supervision, with sensors (1) disposed on the body surface and a data recording unit (12) co-operating with them for operating a data exchange, which device comprises at least one autonomous sensor unit (1 - 3) incorporating transmitter and receiver electrodes (2) and one of the sensors (1), a central transmitter and receiver unit (10) and a portable data recording unit (12), and the central transmitter and receiver unit (10) is placed on the body, the sensor unit (1 - 3) contains a microchip (3) which processes the signals recorded by the sensor (1) into sensor data, the sensor data can be exchanged between the sensor unit (1 - 3) and the central transmitter and receiver unit (10) directly via the skin or tissue and is therefore bodybound, the sensor unit (1 - 3) is disposed on a sensor carrier (4) in the form of a plaster and the microchip (3) comprises a measurement data detection unit (6) connected downstream of the sensor (1) and a transceiver (7) connected to the transmitter and receiver electrodes (2).

2. Device as claimed in claim 1, **characterised in that** the microchip (3) has a battery (8) and a control and monitoring unit (9) for the voltage supply.

3. Device as claimed in claim 1 or 2, **characterised in that** the portable data recording unit is a data logger (12) .

4. Device as claimed in claim 3, **characterised in that** the data transmission between the central transmitter and receiver unit (10) and the data logger (12) takes place by telemetry.

## Revendications

1. Dispositif de surveillance médicale à long terme de personnes destiné à l'assistance médicale à l'aide de capteurs (1) agencés sur la surface du corps ainsi qu'à l'aide d'une unité d'enregistrement de données (12) affectée à ceux-ci pour un échange de données, le dispositif étant constitué d'au moins une unité de détection autonome (1 - 3) qui présente des électrodes d'émission et de réception (2) et un des capteurs (1), d'une unité centrale d'émission et de réception (10) ainsi que d'une unité portable d'enregistrement de données (12), dans lequel l'unité centrale d'émission et de réception (10) est agencée sur le corps, l'unité de détection (1 - 3) contient une micropuce (3) qui transforme les signaux enregistrés par le capteur (1) en données de détection, les données de détection peuvent être échangées entre l'unité de détection (1 - 3) et l'unité centrale d'émission et de réception (10) immédiatement via la peau ou le tissu et, par conséquent, en liaison avec le corps, l'unité de détection (1 - 3) est agencée sur un support de détection (4) qui se présente sous la forme d'un pansement et la micropuce (3) comprend une unité de saisie de données de mesure (6) connectée en aval du capteur (1) ainsi qu'un récepteur-émetteur (7) qui est raccordé aux électrodes d'émission et de réception (2).

2. Dispositif selon la revendication 1, **caractérisé en ce que** la micropuce (3) comprend une pile (8) ainsi qu'une unité de contrôle et de surveillance (9) pour l'alimentation en tension.

3. Dispositif selon la revendication 1 ou 2, **caractérisé en ce que** l'unité portable d'enregistrement de données est constituée d'un enregistreur de données (12).

4. Dispositif selon la revendication 3, **caractérisé en ce que** la transmission de données entre l'unité centrale d'émission et de réception (10) et l'enregistreur de données (12) s'effectue par télémesure.
